# EUROPÄISCHE PATENTANMELDUNG

(11) **EP 4 851 275 A1**
(43) Veröffentlichungstag der Anmeldung: **09.12.2026**
(21) Anmeldenummer: 25181422.4
(22) Anmeldetag: 06.06.2025
(51) Int. Cl.: C12N 1/16, C12N 1/14, C12R 1/645, C12R 1/865

(54) **VERFAHREN ZUR HERSTELLUNG EINES NÄHRMEDIUMS, NÄHRMEDIUM HERGESTELLT NACH DEM VERFAHREN UND VERWENDUNG DES NÄHRMEDIUMS**

(71) Anmelder: Jäckering Research GmbH, 59067 Hamm (DE)
(72) Erfinder: BISCHOFF, Felix, 59368 Werne (DE); ANDREAE-JÄCKERING, Michael, 48155 Münster (DE)
(74) Vertreter: Michalski Hüttermann & Partner mbB

(57) **Zusammenfassung**

Die Erfindung betrifft ein Verfahren zur Herstellung eines Nährmediums (1), umfassend die Schritte: Bereitstellen (S1) eines Gemisches (10) aus Wasser in einem Bereich von ≥ 70 Gew.-% bis ≤ 97 Gew.-% und pflanzlicher Trockenmasse in einem Bereich von ≥ 3 Gew.-% bis ≤ 30 Gew.-%, wobei die Trockenmasse vergärbare Kohlenhydrate, lösliche Ballaststoffe, Proteine, Fette und Asche umfasst; Erhitzen (S2) des Gemisches (10) auf eine Temperatur im Bereich von ≥ 70 bis ≤ 95 °C für einen Zeitraum im Bereich von ≥ 0,5 h bis ≤ 2,5 h; Zugeben (S3) einer Amyloglucosidase und/oder alpha-Amylase (20) zu dem Gemisch (10) und Erhitzen des Gemisches auf eine Temperatur im Bereich von ≥ 55 bis ≤ 85 °C für einen Zeitraum im Bereich von ≥ 0,5 h bis ≤ 8 h; Filtration (S4) des Gemisches (10) mit einer Ultrafiltrationsmembran mit einer Ausschlussgrenze im Bereich von ≥ 50 Dalton bis ≤ 200 Dalton und Isolieren des Filtrats (30) der Filtration (S4) als Nährmedium (1), sowie Produkte daraus und Verwendungen davon.

## Beschreibung

### Technisches Gebiet

Die nachfolgenden Ausführungen betreffen ein Verfahren zur Herstellung eines Nährmediums, ein nach dem Verfahren hergestelltes Nährmedium und die Verwendung des Nährmediums.

### Technischer Hintergrund

Nährmedien werden in der biotechnologischen Industrie, der Lebensmittelproduktion sowie in der mikrobiellen und zellulären Kultivierung eingesetzt. Es ist allgemein bekannt, dass Nährmedien aus einer Kombination von beispielsweise Kohlenstoff- und Stickstoffquellen, Mineralstoffen, Spurenelementen und Vitaminen bestehen, um das Wachstum von Mikroorganismen oder Zellkulturen zu unterstützen. Dabei können sowohl natürliche als auch synthetische Komponenten als Ausgangsstoffe verwendet werden. Fermentationsmedien für Hefen und Pilze basieren häufig auf Zuckerquellen wie Glukose oder Melasse sowie auf organischen Stickstoffquellen wie Hefeextrakt oder Pepton und/oder anorganischen Stickstoffquellen wie Ammoniak bzw. Ammoniumsalzen oder Nitraten. Diese Medien dienen der industriellen Fermentation, der Herstellung von Bioprodukten und der Forschung zur Optimierung mikrobieller Stoffwechselprozesse.

Es ist weiterhin bekannt, dass bestimmte Nährmedien speziell auf die jeweiligen Mikroorganismen oder Zelllinien abgestimmt sein müssen, um eine effiziente Kultivierung zu ermöglichen. Die Zusammensetzung solcher Medien kann erheblichen Einfluss auf die Wachstumsrate und die Produktbildung haben. In der großtechnischen Produktion werden häufig kostengünstige Substrate verwendet, während in der Forschung und Entwicklung synthetische Medien mit genau definierten Inhaltsstoffen bevorzugt sein können.

Nährmedien und deren Herstellung bieten jedoch noch Verbesserungspotential. Insbesondere kann die Herstellung bestehender gut definierter Nährmedien kostenintensiv sein, was eine Anwendung in größeren industriellen Fermentationen erschwert. Ein weiterer Nachteil ist, dass vergleichsweise günstige Medien oft auf Rohstoffen basieren, deren Zusammensetzung natürlichen Schwankungen unterliegt, was zu Variationen in der Fermentationsleistung führen kann.

Es ist daher die Aufgabe der vorliegenden Anmeldung, ein verbessertes Verfahren zur Herstellung eines Nährmediums bereitzustellen. Insbesondere ist es die Aufgabe ein besonders energie- und rohstoffsparendes Verfahren bereitzustellen, sowie ein Nährmedium, das zumindest für bestimmte Anwendungen eine verbesserte Zusammensetzung aufweist.

Diese und weitere Aufgaben werden durch die Merkmale der unabhängigen Ansprüche gelöst. Vorteilhafte Ausgestaltungen sind in den Unteransprüchen, der Beschreibung und den Zeichnungen angegeben. Sofern technisch möglich, können die Lehren der Unteransprüche beliebig mit den Lehren der Haupt- und Unteransprüche kombiniert werden.

### Beschreibung - Technische Lösung

Mit der Erfindung wird ein Verfahren zur Herstellung eines Nährmediums vorgeschlagen, umfassend zumindest die Schritte:
a. Bereitstellen eines Gemisches bestehend aus Wasser in einem Bereich von ≥ 70 Gew.-% bis ≤ 97 Gew.-% und pflanzlicher Trockenmasse in einem Bereich von ≥ 3 Gew.-% bis ≤ 30 Gew.-%, bezogen auf das Gesamtgewicht des Gemisches, wobei die Trockenmasse vergärbare Kohlenhydrate, lösliche Ballaststoffe, Proteine, Fette und Asche umfasst;
b. Erhitzen des Gemisches auf eine Temperatur im Bereich von ≥ 70 bis ≤ 95°C für einen Zeitraum in einem Bereich von ≥ 0,5 h bis ≤ 2,5 h;
c. Zugeben einer Amyloglucosidase und/oder alpha-Amylase zu dem Gemisch und Erhitzen des Gemisches auf eine Temperatur im Bereich von ≥ 55 bis ≤ 85°C für einen Zeitraum in einem Bereich von ≥ 0,5 h bis ≤ 8 h;
d. Filtration des Gemisches mit einer Ultrafiltrationsmembran mit einer Ausschlussgrenze in einem Bereich von ≥ 50 Dalton bis ≤ 200 Dalton und Isolieren des Filtrats der Filtration als Nährmedium.

Nachfolgend werden vorteilige Aspekte erläutert und weiter nachfolgend bevorzugte modifizierte Ausführungsformen beschrieben. Erläuterungen, insbesondere zu Vorteilen und Definitionen von Merkmalen, sind dem Grunde nach beschreibende und bevorzugte, jedoch nicht limitierende Beispiele. Sofern eine Erläuterung limitierend ist, wird dies ausdrücklich erwähnt.

Soweit Ordinalzahlen, zum Beispiel "erste", "zweite", usw., verwendet werden, beispielsweise zur Bezeichnung einer Komponente, eines Elements, eines Verfahrensschritts oder einer Verfahrenshandlung, so sind diese Ordinalzahlen rein zur Differenzierung in der Bezeichnung vorgesehen und zeigen keine Abhängigkeiten oder Reihenfolgen an. Das heißt insbesondere, dass beispielsweise eine Vorrichtung nicht eine "erste Komponente" aufweisen muss, um eine "zweite Komponente" aufzuweisen. Auch kann eine Vorrichtung eine "erste Komponente", sowie eine "dritte Komponente" aufweisen, ohne aber zwangsläufig eine "zweite Komponente" aufzuweisen. Es können auch mehrere Einheiten der gleichen Ordinalzahl vorgesehen sein, also beispielsweise mehrere "erste Komponenten".

Es konnte überraschend gezeigt werden, dass mit dem erfindungsgemäßen Verfahren zur Herstellung eines Nährmediums unter anderem eine Vielzahl der folgenden Vorteile erzielt werden kann. Insbesondere kann erreicht werden, dass das Nährmedium auf besonders ressourcenschonende Weise hergestellt werden kann und direkt aus einem kontinuierlichen Prozess erhalten werden kann. Im vergleich zu Herstellung bekannter Nähmedien kann dabei vorteilhafterweise darauf verzichtet werden, dass das Nähmedium aus verschiedenen Einzelsubstanzen zusammengemischt wird. Dadurch kann vorteilhafterweise auf eine gegebenenfalls aufwändige Isolierung der Einzelsubstanzen verzichtet werden. Trotzdem kann mit dem Verfahren eine gleichbleibende Qualität und Zusammensetzung des Nährmediums erreicht werden, auch wenn die pflanzlichen Ausgangsstoffe natürlichen Schwankungen unterliegen können. Zudem kann das Verfahren so gestaltet werden, dass es sich gut in bestehende Produktionsanlagen integrieren lässt und für industrielle Maßstäbe einfach skalierbar ist. Ferner kann durch das Verfahren erreicht werden, dass Nebenströme der Stärkeherstellung besonders effizient genutzt werden können. Insbesondere kann der Nutzen derartiger Nebenströme auf Anwendungen mit höheren technischen Ansprüchen erweitert werden. Ferner kann erreicht werden, dass mit dem Verfahren vergleichsweise günstige Nähmedien, insbesondere für die Kultivierung von Pilzen in großen Mengen, beispielsweise für die Verwendung in Fleischersatzstoffen, erhalten werden und gegebenenfalls auch Pilze und Hefen gezüchtet werden können, die zuvor überhaupt nicht oder zumindest wirtschaftlich nicht zugänglich waren. Ferner kann mit dem Verfahren vorteilhafterweise erreicht werden, dass auch die neben dem Nährmedium erhaltenen Nebenströme noch weiter verwendet werden können. Durch die gleichzeitige Gewinnung von Flüssigfuttermittel oder von Fraktionen mit definiertem Ballaststoffgehalt kann das Verfahren eine besonders hohe Stoffeffizienz aufweisen.

Gemäß der vorliegenden Erfindung kann unter dem Begriff 'Gemisch' eine physikalische Kombination von zwei oder mehr Substanzen verstanden werden, bei der die einzelnen Bestandteile ihre chemische Identität behalten. Ein Gemisch im Sinne der vorliegenden Erfindung kann sowohl aus festen, flüssigen als auch gelösten Komponenten bestehen. Es kann dabei sowohl homogene Systeme, wie Lösungen, in denen die Bestandteile vollständig gelöst vorliegen umfassen, als auch heterogene Systeme, wie Suspensionen oder Dispersionen, in denen feste Stoffe in einer Flüssigkeit verteilt sind.

Gemäß der vorliegenden Erfindung kann unter dem Begriff 'Trockenmasse' Material verstanden werden, das nach Entfernung von Wasser oder Lösungsmittel aus dem Gemisch zurückbleibt.

Gemäß der vorliegenden Erfindung kann unter dem Begriff ,pflanzliche Trockenmasse' derartige Trockenmasse verstanden werden, die aus pflanzlichen Rohstoffen gewonnen wurde und insbesondere Stärke, Proteinen, Fetten, Ballaststoffen und mineralischen Bestandteilen umfasst.

Gemäß der vorliegenden Erfindung kann unter ,Amyloglucosidase' bzw. ,alpha-Amylase' ein Enzym verstanden werden, das α-1,4- und α-1,6-glykosidische Bindungen in Stärke abbaut. Unter einer Amyloglucosidase kann dabei insbesondere eine Exoamylase verstanden werden, welche vom Ende der Stärkekette her Glucosemoleküle abspaltet. Unter einer alpha-Amylasen kann dabei insbesondere eine Endoamylase verstanden werden, welche Stärkemoleküle innerhalb der der Stärkekette spaltet.

Bevorzugt kann vorgesehen sein, dass in Schritt c) eine Amyloglucosidase verwendet wird. Dadurch kann erreicht werden, dass das Verfahren in Schritt d) weniger anfällig für nicht vollständige Umsatze in Schritt c) ist. Ohne an eine Theorie gebunden zu sein wird davon ausgegangen, dass bei der Verwendung einer Amyloglucosidase Glucose bereits am Anfang der Reaktion in Schritt c) erzeugt wird. Auch bei unvollständigem Umsatz der Stärke kann somit das Reaktionsgemisch in Schritt c) einen bevorzugten Glucoseanteil aufweisen und verbleibende längere Stärkeketten können gegebenenfalls abgetrennt werden.

Alternativ bevorzugt kann vorgesehen sein, dass in Schritt c) eine alpha-Amylase verwendet wird. Dadurch kann vorteilhafterweise erreicht werden, dass die Reaktion in Schritt c) ggf. schneller vonstatten geht. Ohne an eine Theorie gebunden zu sein wird davon ausgegangen, dass beim Umsatz mit alpha-Amylase die Stärke insgesamt schneller gespalten wird, da bei der durch die alpha-Amylase verursachten mittigen Spaltung die entstehenden Teile beide weiter an der Reaktion teilnehmen, wodurch diese beschleunigt werden kann.

Unter einer 'Ultrafiltrationsmembran' im Sinne der vorliegenden Erfindung kann insbesondere eine halbdurchlässige Membran verstanden werden, die zur Trennung von Stoffen auf Basis ihrer Molekülgröße eingesetzt wird. Sie lässt niedermolekulare Substanzen wie Wasser, Salze oder kleine organische Moleküle passieren, während größere Moleküle, wie Proteinagglomerate oder Polysaccharide, zurückgehalten werden. Lösliche Proteine werden hindurch gelassen und können der Stickstoffversorgung der Microorganismen des Nährmediums dienen. Unter einer ,Ausschlussgrenze' kann dabei insbesondere die maximale Molekülgröße verstanden werden, die die Membran noch passieren kann. Sie wird üblicherweise als Molekulargewicht in Dalton (Da) oder Kilodalton (kDa) angegeben und stellt einen Richtwert dar, ab dem Moleküle in signifikanter Weise zurückgehalten werden. Moleküle mit einem höheren Molekulargewicht als die Ausschlussgrenze werden in der Regel nicht durch die Membran hindurchgelassen.

Bevorzugt kann vorgesehen sein, dass das Gemisch bezogen auf das Gesamtgewicht des Gemisches aufweist:
- Wasser in einer Menge im Bereich von ≥ 72,5 Gew.-% bis ≤ 97 Gew.-%, vorzugsweise ≥ 80 Gew.-% bis ≤ 95 Gew.-%, besonders bevorzugt ≥ 88 Gew.-% bis ≤ 92 Gew.-%;
- vergärbare Kohlenhydrate in einer Menge im Bereich von ≥ 1,75 Gew.-% bis ≤ 21,5 Gew.-%, bevorzugt ≥ 4 Gew.-% bis ≤ 12 Gew.-%, besonders bevorzugt ≥ 6 Gew.-% bis ≤ 8 Gew.-%;
- lösliche Ballaststoffe in einer Menge im Bereich von ≥ 0,25 Gew.-% bis ≤ 2,5 Gew.-%, bevorzugt ≥ 0,5 Gew.-% bis ≤2 Gew.-%, besonders bevorzugt ≥ 1 Gew.-% bis ≤ 1,2 Gew.-%;
- Proteine in einer Menge im Bereich von ≥ 0,3 Gew.-% bis ≤ 6 Gew.-%, bevorzugt ≥ 0,5 Gew.-% bis ≤2 Gew.-%, besonders bevorzugt ≥ 0,8 Gew.-% bis ≤ 1,2 Gew.-%;
- Fette in einer Menge im Bereich von ≥ 0,05 Gew.-% bis ≤ 2 Gew.-%, bevorzugt ≥ 0,3 Gew.-% bis ≤ 1 Gew.-%, besonders bevorzugt ≥ 0,6 Gew.-% bis ≤ 0,8 Gew.-%; und/oder
- Asche in einer Menge im Bereich von ≥ 0,05 Gew.-% bis ≤ 1,3 Gew.-%, bevorzugt ≥ 0,1 Gew.-% bis ≤ 0,5 Gew.-%, besonders bevorzugt ≥ 0,15 Gew.-% bis ≤ 0,25 Gew.-%.

Es konnte überraschend gezeigt werden, dass die somit erhaltene Zusammensetzung des Nährmediums besonders gut für das Kultivieren von Mikroorganismen wie Hefen oder Pilzen geeignet ist. Darüber hinaus konnte gezeigt werden, dass die Verwendung eines Gemisches mit dieser Zusammensetzung eine besonders gute Prozessführung mit hoher Effizienz und Ausbeute, insbesondere im Hinblick auf die enzymatische Umsetzung und die Filtration ermöglicht. Zudem konnte gezeigt werden, dass sich durch das Einstellen der Zusammensetzung in diesem Bereich eine gleichbleibende Produktqualität bei variierenden Rohstoffchargen erzielen lässt.

Gemäß der vorliegenden Erfindung können unter vergärbaren Kohlenhydraten insbesondere Monosaccharide, Oligosaccharide und Polysaccharide verstanden werden, die durch Mikroorganismen enzymatisch abgebaut und zur Energiegewinnung verwertet werden können. Bevorzugt umfasst der Begriff Glucose, Fructose, Maltose, Dextrine sowie native oder enzymatisch aufgeschlossene Stärke, insbesondere Weizenstärke.

Gemäß der vorliegenden Erfindung können unter löslichen Ballaststoffen pflanzliche Polysaccharide verstanden werden, die im wässrigen Medium gelöst oder kolloidal verteilt vorliegen und nicht durch menschliche Verdauungsenzyme abgebaut werden. Bevorzugt umfasst der Begriff lösliche Fraktionen wie Arabinoxylane und Arabinogalactane. Gemäß der vorliegenden Erfindung kann unter Arabinoxylanen eine Klasse pflanzlicher Polysaccharide verstanden werden, die hauptsächlich aus einem Rückgrat von β-1,4-verknüpften Xyloseeinheiten bestehen, an die in unterschiedlichem Maße Arabinose-Seitenketten gebunden sind. Arabinoxylane gehören bekannter Weise zu den Hemicellulosen und kommen bevorzugt in den Zellwänden von Getreidepflanzen, insbesondere Weizen, vor. Gemäß der vorliegenden Erfindung kann unter Arabinogalactanen eine Gruppe löslicher, verzweigter Polysaccharide verstanden werden, die aus einem Galactan-Rückgrat mit Seitenketten aus Arabinose bestehen. Arabinogalactane kommen bekannter Weise in verschiedenen pflanzlichen Zellwänden vor, unter anderem in Weizen und anderen Getreidearten. Im Sinne der vorliegenden Erfindung sind unter dem Begriff "Arabinoxylane" stets auch "Arabinogalactane" mitumfasst, sofern nicht ausdrücklich etwas anderes angegeben ist.

Gemäß der vorliegenden Erfindung kann unter Proteinen die Gesamtheit der im pflanzlichen Rohmaterial enthaltenen Eiweißstoffe verstanden werden, wobei insbesondere nicht-Gluten-Proteine wie Albumine und Globuline bevorzugt sind. Diese Proteine können ganz oder teilweise wasserlöslich oder dispergierbar sein und können bevorzugt aus Weizen stammen.

Gemäß der vorliegenden Erfindung können unter Fetten pflanzliche Lipide verstanden werden, die sowohl freies Öl, insbesondere Weizenkeimöl, als auch an Proteine gebundene Fettsäuren umfassen.

Gemäß der vorliegenden Erfindung kann unter Asche die mineralische Rückstandssubstanz verstanden werden, die nach vollständiger Verbrennung der organischen Bestandteile verbleibt. Die Asche kann insbesondere Calciumoxid, Kaliumoxid, Magnesiumoxid, Phosphorpentoxid, Siliciumdioxid, Eisen(III)-oxid und Natriumcarbonat enthalten.

Bevorzugt kann vorgesehen sein, dass im bereitgestellten Gemisch
- die vergärbaren Kohlenhydrate Stärke umfassen, bevorzugt Weizenstärke, wobei die Weizenstärke insbesondere Amylose und Amylopektin umfasst;
- die löslichen Ballaststoffe ausgewählt sind aus der Gruppe bestehend aus Arabinoxylanen und Arabinogalactanen;
- die Proteine nicht-Gluten Proteine umfassen, bevorzugt nicht-Gluten Weizenproteine, wobei die nicht-Gluten Weizenproteine insbesondere ausgewählt sind aus der Gruppe bestehend aus Albuminen und Globulinen;
- die Fette ausgewählt sind aus der Gruppe bestehend aus Weizenkeimöl und an Proteine gebundenen Fettsäuren; und/oder
- die Asche mineralische Bestandteile umfasst, insbesondere ausgewählt aus der Gruppe bestehend aus Calciumoxid, Kaliumoxid, Magnesiumoxid, Phosphorpentoxid, Siliciumdioxid, Eisen(III)-oxid und Natriumcarbonat.

Es konnte überraschend gezeigt werden, diese Bestandteile besonders gut für das Verfahren geeignet sind und sich eine besonders gute Prozessführung mit hoher Effizienz und Ausbeute des Nährmittels erreichen lässt. Ferner konnte gezeigt werden, dass sich eine derartige Zusammensetzung besonders effizient aus Nebenproduktströmen einer Weizenstärkeproduktion erhalten lässt.

Bevorzugt kann vorgesehen sein, dass die löslichen Ballaststoffe im bereitgestellten Gemisch Arabinoxylan aufweisen, wobei das Arabinoxylan ein gewichtsgemitteltes Molekulargewicht Mw in einem Bereich von ≥ 300 kDa bis ≤ 500 kDa aufweist oder in einem Bereich von ≥ 10 kDa bis ≤ 100 kDa. Bevorzugt kann vorgesehen sein, dass die Ausschlussgrenze der Ultrafiltrationsmembran kleiner ist als das gewichtsgemitteltes Molekulargewicht Mw des Arabinoxylan.

Im Sinne der vorliegenden Erfindung bezeichnet der Begriff ,gewichtsgemitteltes Molekulargewicht' (Mw) das Molekulargewicht, bei dem die Massenanteile der jeweiligen Molekülarten berücksichtigt werden. Es wird nach folgender Formel bestimmt: $Mw = \sum \left({ω}_{i}\times {M}_{i}\right)$

Dabei ist: Mw das gewichtsgemittelte Molekulargewicht, *Mi* das Molekulargewicht des jeweiligen Moleküls, *ωᵢ* der Massenanteils des jeweiligen Moleküls.

Das gewichtsgemittelte Molekulargewicht kann gemäß DIN 55672-1:2016-04 mittels Größenausschlusschromatographie (GPC) bestimmt werden.

In vorteilhafter Weise kann dadurch erreicht werden, dass durch die gezielte Einstellung des gewichtsgemittelten Molekulargewichts des Arabinoxylans im bereitgestellten Gemisch eine besonders vorteilhafte Steuerung der funktionellen Eigenschaften des Nährmediums möglich sein kann. Insbesondere kann erreicht werden, dass bei der Verwendung von Arabinoxylanen mit einem gewichtsgemittelten Molekulargewichts im Bereich von ≥ 300 kDa bis ≤ 500 kDa das erhaltene Nährmedium einen höheren Anteil an C6-Fraktionen und einen geringeren Anteil an Pentosanen aufweisen kann. Zudem kann bei der Verwendung derartiger Arabinoxylane im Gemisch ein abgetrenntes Futtermittel einen größeren Proteingehalt und geringeren Ballaststoffgehalt aufweisen, wobei das Arabinoxylan zusätzlich aus dem Futtermittel isoliert werden kann und als separate Zusammensetzung aufbereitet werden kann. Dadurch kann die Verwertung der Gemischbestandteile besonders effizient sein. Bei der Verwendung von Arabinoxylanen mit einem gewichtsgemittelten Molekulargewichts im Bereich von ≥10 kDa bis ≤ 100 kDa kann das erhaltene Nährmedium indes einfacher herstellbar sein und eine Futtermittelzusammensetzung ohne weitere Aufbereitung verwertbar sein.

Bevorzugt kann vorgesehen sein, dass das Gemisch aus Nebenströmen einer Weizenstärkeproduktion stammt.

Es konnte überraschend gezeigt werden, dass Nebenströme aus der Weizenstärkeproduktion eine besonders geeignete Rohstoffquelle für die Herstellung des Nährmediums darstellen. Insbesondere kann erreicht werden, dass durch die Verwendung solcher Nebenströme ein ökonomisch und ökologisch vorteilhafter Herstellungsweg erschlossen wird, bei dem bestehende Ressourcen effizient genutzt werden.

Bevorzugt kann vorgesehen sein, dass das Gemisch mindestens einen, vorzugsweise alle der folgenden Nebenströme aus einer Weizenstärkeproduktion umfasst, bezogen auf das Gesamtgewicht des Gemisches:
- Prozesswasser in einer Menge im Bereich von ≥ 0 Gew.-% bis ≤ 50 Gew.-%, bevorzugt ≥ 15 Gew.-% bis ≤ 40 Gew.-%, besonders bevorzugt ≥ 25 Gew.-% bis ≤ 30 Gew.-%;
- C-Stärke-Fraktion in einer Menge im Bereich von ≥ 0 Gew.-% bis ≤ 50 Gew.-%, bevorzugt ≥ 2,5 Gew.-% bis ≤ 20 Gew.-%, besonders bevorzugt ≥ 5 Gew.-% bis ≤ 10 Gew.-%; und
- Pentosan-Fraktion in einer Menge im Bereich von ≥ 50 Gew.-% bis ≤ 100 Gew.-%, bevorzugt ≥ 60 Gew.-% bis ≤ 85 Gew.-%, besonders bevorzugt ≥ 65 Gew.-% bis ≤ 70 Gew.-%.

Dadurch lässt sich die gewünschte Zusammensetzung des Gemisches besonders effizient erreichen.

Unter 'Prozesswasser' ist im Sinne der vorliegenden Erfindung das Wasser zu verstehen, welches in der Weizenstärkeproduktion als flüssiger Nebenstrom bei verschiedenen Wasch- und Trennschritten anfällt. Es enthält in gelöster oder suspendierter Form geringe Mengen an Bestandteilen aus der Weizenkornmatrix, insbesondere niedermolekulare Kohlenhydrate, lösliche Ballaststoffe, Proteine, Fette und Mineralstoffe.

Die 'C-Stärke-Fraktion' ist im Sinne der vorliegenden Erfindung insbesondere ein Nebenprodukt der Weizenstärkegewinnung, welches nach Abtrennung der hochwertigen A- und B-Stärken zurückbleibt. Sie enthält neben Reststärke vor allem Protein, Ballaststoffe und weitere begleitende Kornbestandteile in suspendierter Form.

Die ,Pentosan-Fraktion' im Sinne der vorliegenden Erfindung umfasst Pentosane, die während des Nassprozesses aus dem Weizen extrahiert werden. Die Pentosan-Fraktion entsteht als flüssige Phase nach der Trennung von A-, B- und C-Stärke sowie Gluten.

Bevorzugt kann vorgesehen sein, dass das Prozesswasser bezogen auf das Gesamtgewicht des Prozesswassers aufweist:
- Wasser in einer Menge im Bereich von ≥ 85 Gew.-% bis ≤ 99 Gew.-%, bevorzugt ≥ 90 Gew.-% bis ≤ 98 Gew.-%;
- vergärbare Kohlenhydrate in einer Menge von ≥ 1 Gew.-% bis ≤ 10 Gew.-%, bevorzugt ≥ 2 Gew.-% bis ≤ 5 Gew.-%;
- lösliche Ballaststoffe in einer Menge von ≥ 0 Gew.-% bis ≤ 1 Gew.-%, bevorzugt ≥ 0,1 Gew.-% bis ≤ 0,5 Gew.-%;
- Protein in einer Menge von ≥ 0,1 Gew.-% bis ≤ 10 Gew.-%, bevorzugt ≥0,1 Gew.-% bis ≤ 0,5 Gew.-%;
- Fett in einer Menge von ≥ 0 Gew.-% bis ≤ 1 Gew.-%, bevorzugt ≥ 0,1 Gew.-% bis ≤ 0,5 Gew.-%; und/oder
- Asche in einer Menge von ≥ 0 Gew.-% bis ≤ 2 Gew.-%, bevorzugt ≥ 0,01 Gew.-% bis ≤ 0,2 Gew.-%.

Dadurch lässt sich die gewünschte Zusammensetzung des Gemisches besonders gut einstellen.

Bevorzugt kann vorgesehen sein, dass die C-Stärke-Fraktion bezogen auf das Gesamtgewicht der C-Stärke-Fraktion aufweist:
- Wasser in einer Menge im Bereich von ≥ 60 Gew.-% bis ≤ 90 Gew.-%, bevorzugt ≥ 70 Gew.-% bis ≤ 80 Gew.-%;
- vergärbare Kohlenhydrate in einer Menge von ≥ 7 Gew.-% bis ≤ 35 Gew.-%, bevorzugt ≥ 15 Gew.-% bis ≤ 25 Gew.-%;
- lösliche Ballaststoffe in einer Menge von ≥ 0,2 Gew.-% bis ≤ 2,5 Gew.-%, bevorzugt ≥ 0,5 Gew.-% bis ≤ 1,5 Gew.-%;
- Protein in einer Menge von ≥ 0,2 Gew.-% bis ≤ 3 Gew.-%, bevorzugt ≥ 0,5 Gew.-% bis ≤ 1,5 Gew.-%;
- Fett in einer Menge von ≥ 0 Gew.-% bis ≤ 1 Gew.-%, bevorzugt ≥ 0,1 Gew.-% bis ≤ 0,5 Gew.-%; und/oder
- Asche in einer Menge von ≥ 0 Gew.-% bis ≤ 2 Gew.-%, bevorzugt ≥ 0,1 Gew.-% bis ≤ 1 Gew.-%.

Es konnte genezeigt werden, dass sich eine C-Stärke-Fraktion mit dieser Zusammensetzung besonders gut aus einem bestehenden Prozess zur Weizenstärkeproduktion isolieren lässt. Ferner konnte gezeigt werden, dass sich mit dieser Zusammensetzung die gewünschte Zusammensetzung des Gemisches besonders gut einstellen lässt.

Bevorzugt kann vorgesehen sein, dass die Pentosan-Fraktion bezogen auf das Gesamtgewicht der Pentosan-Fraktion aufweist:
- Wasser in einer Menge im Bereich von ≥ 85 Gew.-% bis ≤ 95 Gew.-%, bevorzugt ≥ 88 Gew.-% bis ≤ 92 Gew.-%;
- vergärbare Kohlenhydrate in einer Menge von ≥ 2,5 Gew.-% bis ≤ 8 Gew.-%, bevorzugt ≥ 5 Gew.-% bis ≤ 7 Gew.-%;
- lösliche Ballaststoffe in einer Menge von ≥ 0,5 Gew.-% bis ≤ 2,5 Gew.-%, bevorzugt ≥ 1 Gew.-% bis ≤ 2 Gew.-%;
- Protein in einer Menge von ≥ 0,5 Gew.-% bis ≤ 2 Gew.-%, bevorzugt ≥ 1 Gew.-% bis ≤ 1,5 Gew.-%;
- Fett in einer Menge von ≥ 0,1 Gew.-% bis ≤ 2 Gew.-%, bevorzugt ≥ 0,5 Gew.-% bis ≤ 1 Gew.-%; und/oder
- Asche in einer Menge von ≥ 0,1 Gew.-% bis ≤ 0,6 Gew.-%, bevorzugt ≥ 0,2 Gew.-% bis ≤ 0,4 Gew.-%.

Es konnte gezeigt werden, dass sich eine Pentosan-Fraktion mit dieser Zusammensetzung besonders gut aus einem bestehenden Prozess zur Weizenstärkeproduktion isolieren lässt. Ferner konnte gezeigt werden, dass sich mit dieser Zusammensetzung die gewünschte Zusammensetzung des Gemisches gut einstellen lässt.

Bevorzugt kann vorgesehen sein, dass das Filtrat aus Schritt d) in einem Schritt e) mittels eines Vakuumverdampfers konzentriert wird.

In vorteilhafter Weise kann erreicht werden, dass durch die zusätzliche Konzentration des Filtrats mittels eines Vakuumverdampfers der Gehalt an Trockensubstanz im Nährmedium gezielt eingestellt werden kann. Dadurch lässt sich insbesondere die Lagerfähigkeit verbessern und das Volumen für Transport und Handhabung reduzieren. Zudem kann durch die Konzentration der Nährstoffgehalt des Mediums erhöht und somit an spezifische Anforderungen verschiedener Anwendungen angepasst werden.

Bevorzugt kann vorgesehen sein, dass Schritt e) bei einer Temperatur im Bereich von ≥ 60 °C bis ≤ 90 °C, bevorzugt ≥ 75°C bis ≤ 85 °C, und/oder einem Druck im Bereich von ≥ 50 mbar bis ≤ 300 mbar, bevorzugt ≥ 150 mbar bis ≤ 250 mbar durchgeführt wird, wobei der Prozess bevorzugt kontinuierlich verläuft.

Dadurch kann vorteilhafterweise erreicht werden, dass durch die Durchführung des Konzentrationsschritts e) bei den angegebenen Temperaturen und Drücken eine besonders schonende Eindampfung des Filtrats möglich ist. Dadurch können hitzeempfindliche Bestandteile wie Zucker oder funktionelle Ballaststoffe geschont werden. Ferner ermöglicht der kontinuierliche Betrieb eine energieeffiziente Prozessführung und eine gleichmäßige Produktqualität über längere Zeiträume hinweg.

Bevorzugt kann vorgesehen sein, dass in Schritt e) das Filtrat aus Schritt d) auf einen Gehalt an Trockensubstanz in einem Bereich von ≥ 20 Gew.-% bis ≤ 70 Gew.-% konzentriert wird, bevorzugt ≥ 30 Gew.-% bis ≤ 60 Gew.-%, besonders bevorzugt ≥ 45 Gew.-% bis ≤ 55 Gew.-%.

Es konnte gezeigt werden, dass sich durch die Einstellung des Trockensubstanzgehalts in diesem Bereich die Eigenschaften des Nährmediums gezielt beeinflussen lassen. Insbesondere kann erreicht werden, dass die Konzentration hoch genug ist, um das Nährmedium effizient zu lagern und zu transportieren. Zudem kann durch die bevorzugten Bereiche eine stabile und homogene Zusammensetzung des Produkts gewährleistet werden.

Bevorzugt kann vorgesehen sein, dass in Schritt b) die Bestandteile des Gemisches zumindest teilweise hydrolysiert werden. Dies lässt sich durch die beschriebenen Prozessparameter in Schritt b) und besonders durch die weiter bevorzugten Prozessparameter erreichen.

Dadurch kann in vorteilhafter Weise erreicht werden, dass durch die zumindest teilweise Hydrolyse der Bestandteile des Gemisches in Schritt b) eine verbesserte Aufschließung der Nährstoffkomponenten erreicht werden kann. Insbesondere kann dadurch die nachfolgende enzymatische Umsetzung erleichtert und die Ausbeute an vergärbaren Zuckern erhöht werden. Dies trägt zu einer verbesserten Effizienz des Gesamtprozesses bei.

Bevorzugt kann vorgesehen sein, dass in Schritt b) das Gemisch auf eine Temperatur im Bereich von ≥ 75 °C bis ≤ 92 °C, bevorzugt ≥ 80 °C bis ≤ 90 °C, erhitzt wird.

Bevorzugt kann vorgesehen sein, dass in Schritt b) das Gemisch für einen Zeitraum im Bereich von ≥ 0,75 Stunden bis ≤ 2 Stunden, bevorzugt ≥ 1 Stunde bis ≤ 1,5 Stunden, erhitzt wird.

In vorteilhafter Weise kann erreicht, dass durch das Erhitzen des Gemisches in diesem Temperaturbereich und für diese Dauer eine effektive Denaturierung von Proteinen sowie eine thermische Aufschließung von Polysacchariden ermöglicht wird. Dadurch kann die Verfügbarkeit der Substrate für die anschließende enzymatische Umsetzung verbessert und gleichzeitig eine mikrobielle Stabilisierung des Gemisches erreicht werden.

Bevorzugt kann vorgesehen sein, dass in Schritt b) agglomeriertes Protein vor Schritt c) durch mechanische Separation aus dem Gemisch abgetrennt wird, bevorzugt durch Filtration oder Dekanterzentrifuge.

Es konnte gezeigt werden, dass durch die Abtrennung von agglomeriertem Protein vor dem enzymatischen Umsetzungsschritt c), im bereitgestellten Gemisch vorhandene lösliche Ballaststoffe in Schritt d) als Flüssigfuttermittel aus dem Retentat der Filtration isoliert werden können.

In einer alternativ bevorzugen Ausgestaltung kann vorgesehen sein, dass in Schritt b) agglomeriertes Protein nicht vor Schritt c) oder d) aus dem Gemisch abgetrennt wird.

Es konnte gezeigt werden, dass durch das Vermeiden einer Abtrennung des zuvor agglomerierten Proteins die Effizienz der Umsetzung und der darauf folgenden Filtration gesteigert werden kann. Ohne an eine Theorie gebunden zu sein wird davon ausgegangen, dass die agglomerierten Proteine die Ultrafiltrationsmembran schützen, so dass sich diese weniger zusetzt.

Bevorzugt kann vorgesehen sein, dass in Schritt c) Bestandteile des Gemisches zumindest teilweise verzuckert werden. Dies lässt sich durch die beschriebenen Prozessparameter in Schritt c) und besonders durch die weiter bevorzugten Prozessparameter erreichen.

Es konnte gezeigt werden, dass durch die zumindest teilweise Verzuckerung der Bestandteile in Schritt c) ein hoher Anteil an niedermolekularen Kohlenhydraten erzeugt werden kann, die als leicht verfügbare Energiequelle für Mikroorganismen dienen. Dies trägt wesentlich zur Nährwertdichte und zur Funktionalität des so erhaltenen Nährmediums bei.

Bevorzugt kann vorgesehen sein, dass in Schritt c) Amyloglucosidase und/oder alpha-Amylase in einer Menge im Bereich von ≥ 0,05 Gew.-% bis ≤ 0,5 Gew.-%, bezogen auf das Gesamtgewicht des Gemisches hinzugefügt wird.

Bevorzugt kann vorgesehen sein, dass in Schritt c) das Gemisch auf eine Temperatur im Bereich von ≥ 55 °C bis ≤ 85 °C, bevorzugt ≥ 60 °C bis ≤ 75 °C, erhitzt wird.

Bevorzugt kann vorgesehen sein, dass in Schritt c) das Gemisch für einen Zeitraum im Bereich von ≥ 0,5 Stunden bis ≤ 8 Stunden, bevorzugt ≥ 1 Stunde bis ≤ 3 Stunden, erhitzt wird.

Es konnte gezeigt werden, dass durch die Zugabe von Amyloglucosidase und/oder alpha-Amylase in dem angegebenen Mengenbereich das Fahren der Reaktion in diesem Temperaturbereich und für diese Dauer eine besonders effiziente enzymatische Umwandlung von Stärke ermöglicht wird, ohne dass es zu einer Überdosierung oder unnötigen Enzymkosten kommt. Der definierte Bereich erlaubt eine wirtschaftliche und gleichzeitig leistungsfähige Prozessführung.

Bevorzugt kann vorgesehen sein, dass in Schritt d) im Gemisch enthaltenes Protein aus dem Gemisch zumindest teilweise entfernt wird und im Retentat der Ultrafiltration verbleibt.

Es konnte gezeigt werden, dass durch das Zurückhalten von Protein im Retentat während der Ultrafiltration in Schritt d) das Filtrat als Nährmedium eine reduzierte Proteinanteil aufweist. Dies kann insbesondere bei Anwendungen vorteilhaft sein, bei denen eine definierte Kohlenhydratmatrix ohne hohe Proteinanteile erwünscht ist. Gleichzeitig kann das zurückgehaltene Protein gezielt für weitere Anwendungen genutzt werden.

Bevorzugt kann vorgesehen sein, dass in Schritt d) die Ultrafiltrationsmembran eine Ausschlussgrenze im Bereich von ≥ 75 kDa bis ≤ 150 kDa, bevorzugt ≥ 80 kDa bis ≤ 120 kDa aufweist.

Dadurch kann erreicht werden, dass Bestandteile wie insbesondere Proteine zurückgehalten werden, während niedermolekulare Zucker und gelöste Nährstoffe in das Filtrat übergehen. Dadurch kann eine gezielte Fraktionierung des Gemisches erreicht und die gewünschte Zusammensetzung des Nährmediums eingestellt werden.

Bevorzugt kann vorgesehen sein, dass die Ultrafiltration in Schritt d) bei einem Transmembrandruck im Bereich von ≥ 1 bar bis ≤ 4 bar, bevorzugt ≥ 1,5 bar bis ≤ 3 bar durchgeführt wird.

Es konnte überraschend gezeigt werden, dass die Durchführung der Ultrafiltration bei einem Transmembrandruck im angegebenen Bereich zu einer besonders effizienten Filtration mit hoher Flussrate und gleichzeitig geringer Membranbelastung führt. Dadurch kann eine stabile Prozessführung mit langen Standzeiten der Membran erzielt und gleichzeitig eine gleichmäßige Produktqualität gewährleistet werden. Ohne an eine Theorie gebunden zu sein, wird davon ausgegangen, dass mit diesen Parametern im Gemisch vorhandene Flocken aus Protein eine Deckschicht auf der Membran bilden, welche vor Verblockung der Membran durch feinere Bestandteile schützen kann. Dadurch können deutlich höhere Standzeiten erreicht werden.

Bevorzugt kann vorgesehen sein, dass in Schritt d) das Retentat der Filtration als Flüssigfuttermittel isoliert wird.

In vorteilhafter Weise kann dadurch erreicht, dass das in Schritt d) anfallende Retentat eine nährstoffreiche Zusammensetzung aufweist, die sich besonders gut zur Verwendung als Flüssigfuttermittel eignet. Durch die gezielte Isolierung dieses Nebenstroms kann eine vollständige Nutzung des Ausgangsmaterials erreicht und die Effizienz des Verfahrens deutlich verbessert werden.

In einer ersten alternativen bevorzugten Ausgestaltung kann vorgesehen sein, dass die löslichen Ballaststoffe im bereitgestellten Gemisch Arabinoxylan aufweisen, wobei das Arabinoxylan ein gewichtsgemitteltes Molekulargewicht Mw in einem Bereich von ≥ 10 kDa bis ≤ 100 kDa aufweist und in Schritt d) das Retentat der Filtration als Flüssigfuttermittel isoliert wird.

Es konnte gezeigt werden, dass Arabinoxylan mit einem gewichtsgemittelten Molekulargewicht im angegebenen Bereich besonders gut im Retentat zurückgehalten wird und so gezielt in das Flüssigfuttermittel überführt werden kann. Das gewichtsgemittelte Molekulargewichts des Arabinoxylans kann in bekannter Weise beispielsweise durch Anpassungen in der Weizenstärkeproduktion eingestellt werden. Ferner kann entsprechendes Arabinoxylan besonders in Flüssigfuttermitteln verwendet werden. Diese Fraktion wirkt sich positiv auf die Futterwertigkeit aus, da lösliche Ballaststoffe wie Arabinoxylane prebiotische Eigenschaften besitzen und die Verdaulichkeit unterstützen können.

In einer zweiten alternativen bevorzugten Ausgestaltung kann vorgesehen sein, dass die löslichen Ballaststoffe im bereitgestellten Gemisch Arabinoxylan aufweisen, wobei das Arabinoxylan ein gewichtsgemitteltes Molekulargewicht Mw in einem Bereich von ≥ 300 kDa bis ≤ 500 kDa aufweist und in Schritt d) das Retentat der Filtration als Flüssigfuttermittel isoliert wird.

Es konnte gezeigt werden, dass Arabinoxylan mit einem gewichtsgemittelten Molekulargewicht im Bereich von ≥ 300 kDa bis ≤ 500 kDa besonders effektiv im Retentat zurückgehalten wird. Dadurch kann eine Ultrafiltrationsmembran mit besonders großer Ausschlussgrenze genutzt werden, wodurch sich ein höherer Durchsatz der Filtration ergeben kann. Das gewichtsgemittelte Molekulargewichts des Arabinoxylans kann in bekannter Weise beispielsweise durch Anpassungen in der Weizenstärkeproduktion eingestellt werden. Ferner kann dadurch erreicht werden, dass aus dem Retentat Arabinoxylan weiter isoliert werden kann, so dass sich eine Abtrennung einer Arabinoxylanzusammensetzung ergeben kann.

Bevorzugt kann in der zweiten alternativen Ausgestaltung vorgesehen sein, dass in Schritt b) agglomeriertes Protein vor Schritt c) durch mechanische Separation aus dem Gemisch abgetrennt wird, bevorzugt durch Filtration oder Dekanterzentrifuge. Dadurch kann das Arabinoxylan besonders einfach abgetrennt werden.

Bevorzugt kann in der zweiten alternativen Ausgestaltung vorgesehen sein, dass das Isolieren des Retentats der Filtration als Flüssigfuttermittel einen Schritt d2) umfasst, in dem ein weiterer mechanischer Separationsschritt erfolgt, vorzugsweise eine Filtration, wobei das Retentat von Schritt d2) als das Flüssigfuttermittel isoliert wird.

Es konnte gezeigt werden, dass durch die zusätzliche Durchführung eines mechanischen Separationsschritts in Schritt d2) in dieser Ausgestaltung eine gezieltere Auftrennung der Inhaltsstoffe des ursprünglichen Retentats möglich ist. Dadurch kann die Zusammensetzung des erhaltenen Flüssigfuttermittels weiter verbessert und an spezifische Anforderungen angepasst werden, beispielsweise hinsichtlich Proteingehalts.

Bevorzugt kann vorgesehen sein, dass in Schritt d2) Protein des in Schritt d) erhaltenen Retentats der Filtration in dem Retentat des weiteren mechanischen Separationsschrittes von Schritt d2) verbleibt.

Bevorzugt kann vorgesehen sein, dass in Schritt d2) das Filtrat des weiteren mechanischen Separationsschrittes isoliert wird

Es konnte gezeigt werden, dass durch die gezielte Rückhaltung von Protein im Retentat von Schritt d2) das Flüssigfuttermittel eine verbesserte Zusammensetzung aufweisen kann und zudem die Isolation und Aufarbeitung weiterer Bestandteile ermöglicht wird.

Bevorzugt kann vorgesehen sein, dass in Schritt d2) das isolierte Filtrat des weiteren mechanischen Separationsschrittes C6-Zucker und Arabinoxylan mit einem gewichtsgemittelten Molekulargewicht Mw in einem Bereich von ≥ 300 kDa bis ≤ 500 kDa aufweist.

Diese Kombination eignet sich besonders für weiterverarbeitbare Fraktionen, beispielsweise zur Herstellung funktioneller Ballaststoffprodukte oder als prebiotisch wirksame Zusatzstoffe.

Bevorzugt kann vorgesehen sein, dass wobei in Schritt d2) das isolierte Filtrat des weiteren mechanischen Separationsschrittes in einem weiteren Schritt d3) mit einer Ultrafiltrationsmembran mit einer Ausschlussgrenze in einem Bereich von ≥ 50 Dalton bis ≤ 200 Dalton im Diafiltrationsmodus filtriert wird und das Retentat als Arabinoxylanzusammensetzung isoliert wird.

Es konnte gezeigt werden, dass mit diesen Parametern eine besonders reine Arabinoxylanzusammensetzung gewonnen werden kann.

Bevorzugt kann vorgesehen sein, dass die löslichen Ballaststoffe im bereitgestellten Gemisch Arabinoxylan aufweisen, wobei das Arabinoxylan ein gewichtsgemitteltes Molekulargewicht Mw in einem Bereich von ≥ 300 kDa bis ≤ 500 kDa aufweist und in Schritt d) das Retentat der Filtration als Flüssigfuttermittel isoliert wird,
wobei das das Isolieren des Retentats der Filtration als Flüssigfuttermittel einen Schritt d2) umfasst, in dem ein weiterer mechanischer Separationsschritt erfolgt, vorzugsweise eine Filtration, wobei das Retentat von Schritt d2) als das Flüssigfuttermittel isoliert wird und das Filtrat von Schritt d2) isoliert wird,
wobei das isolierte Filtrat von Schritt d2) des weiteren mechanischen Separationsschrittes in einem weiteren Schritt d3) mit einer Ultrafiltrationsmembran mit einer Ausschlussgrenze in einem Bereich von ≥ 50 Dalton bis ≤ 200 Dalton im Diafiltrationsmodus filtriert wird und das Retentat als Arabinoxylanzusammensetzung isoliert wird.

In vorteilhafter Weise kann erreicht, dass durch die zusätzliche Durchführung eines mechanischen Separationsschritts in Schritt d2) in dieser Ausgestaltung eine gezieltere Auftrennung der Inhaltsstoffe des ursprünglichen Retentats möglich ist und die Zusammensetzung des erhaltenen Flüssigfuttermittels weiter verbessert und an spezifische Anforderungen angepasst werden. Es konnte gezeigt werden, dass durch die gezielte Rückhaltung von Protein im Retentat von Schritt d2) das Flüssigfuttermittel eine verbesserte Zusammensetzung aufweisen kann und zudem die Isolation und Aufarbeitung zur Herstellung funktioneller Ballaststoffprodukte erlaubt.

Bevorzugt kann vorgesehen sein, dass das isolierte Retentat aus Schritt d3) in einem Schritt d4) getrocknet wird, vorzugsweise bei einer Temperatur in einem Bereich von ≥ 70 °C bis ≤ 90 °C.

Es konnte gezeigt werden, dass durch das Trocknen des in Schritt d3) isolierten Retentats in einem definierten Temperaturbereich von ≥ 70 °C bis ≤ 90 °C eine stabile, lagerfähige Arabinoxylanzusammensetzung erhalten werden kann. Die gewählte Temperaturführung erlaubt dabei eine schonende Trocknung, bei der die funktionellen Eigenschaften der Polysaccharide weitgehend erhalten bleiben.

Mit der Erfindung wird ferner ein Nährmedium vorgeschlagen, welches nach dem erfindungsgemäßem Verfahren hergestellt wurde.

Es konnte überraschend gezeigt werden, dass das nach dem erfindungsgemäßen Verfahren hergestelltes Nährmedium eine Zusammensetzung aufweist, die sich besonders gut für die Kultivierung verschiedener Mikroorganismen eignet. Insbesondere kann durch das Verfahren ein Nährmedium bereitgestellt werden, das effizient kostengünstigen aus natürlichen Rohstoffquellen stammt und dennoch eine konstante, definierte Qualität aufweist.

Mit der Erfindung wird ein Nährmedium vorgeschlagen, wobei das Nährmedium bezogen auf das Gesamtgewicht der Trockenmasse des Nährmediums aufweist:
- eine C6-Zuckerfraktion in einer Menge im Bereich von ≥ 50 Gew.-% bis ≤ 90 Gew.-%, bevorzugt ≥ 65 Gew.-% bis ≤ 80 Gew.-%,
- Pentosane in einer Menge im Bereich von ≥ 0 Gew.-% bis ≤ 20 Gew.-%, bevorzugt ≥ 1 Gew.-% bis ≤ 12 Gew.-%,
- Fette in einer Menge im Bereich von ≥ 0,1 Gew.-% bis ≤ 15 Gew.-%, bevorzugt ≥ 5 Gew.-% bis ≤ 12 Gew.-%, und
- Asche in einer Menge im Bereich von ≥ 0,5 Gew.-% bis ≤ 10 Gew.-%, bevorzugt ≥ 1 Gew.-% bis ≤ 5 Gew.-%.

Es konnte überraschend gezeigt werden, dass ein Nährmedium mit dieser Zusammensetzung eine besonders gute Nährstoffbasis für das Wachstum von Mikroorganismen wie Pilzen und Hefen bietet. Ferner konnte gezeigt werden, dass sich diese Zusammensetzung besonders gut mit dem vorliegenden Verfahren herstellen lässt.

Bevorzugt kann vorgesehen sein, dass die C6-Zuckerfraktion ausgewählt ist aus der Gruppe bestehend aus Glucose, Fructose, Maltose und/oder Maltodextrinen; und/oder die Pentosane ausgewählt sind aus der Gruppe bestehend aus Arabinoxylanen und Arabinogalactanen.

Bevorzugt kann vorgesehen sein, dass das Arabinoxylan in dem Verfahren ein gewichtsgemitteltes Molekulargewicht Mw in einem Bereich von ≥ 10 kDa bis ≤ 100 kDa aufweist und in Schritt d) das Retentat der Filtration als Flüssigfuttermittel isoliert wurde, und das Nährmedium bezogen auf das Gesamtgewicht der Trockenmasse des Nährmediums aufweist:
- eine C6-Zuckerfraktion in einer Menge im Bereich von ≥ 60 Gew.-% bis ≤ 75 Gew.-%, bevorzugt ≥ 65 Gew.-% bis ≤70 Gew.-%,
- Pentosane in einer Menge im Bereich von ≥ 5 Gew.-% bis ≤15.-%, bevorzugt ≥ 10 Gew.-% bis ≤ 12 Gew.-%,
- Fette in einer Menge im Bereich von ≥ 5 Gew.-% bis ≤ 15 Gew.-%, bevorzugt ≥ 10 Gew.-% bis ≤ 12 Gew.-%, und
- Asche in einer Menge im Bereich von ≥ 0,5 Gew.-% bis ≤5.-%, bevorzugt ≥ 1 Gew.-% bis ≤ 2 Gew.-%.

In vorteilhafter Weise kann dadurch erreicht werden, dass ein Nährmedium mit Arabinoxylan mit einem gewichtsgemittelten Molekulargewicht Mw im Bereich von ≥ 10 kDa bis ≤ 100 kDa besonders gut mit der vorbeschriebenen Zusammensetzung herstellbar ist.

Bevorzugt kann vorgesehen sein, dass das Arabinoxylan in dem Verfahren ein gewichtsgemitteltes Molekulargewicht in einem Bereich von ≥ 300 kDa bis ≤ 500 kDa aufweist und in Schritt d) das Retentat der Filtration als Flüssigfuttermittel isoliert wurde, und das Nährmedium bezogen auf das Gesamtgewicht der Trockenmasse des Nährmediums aufweist:
- eine C6-Zuckerfraktion in einer Menge im Bereich von ≥ 70 Gew.-% bis ≤ 90 Gew.-%, bevorzugt ≥ 75 Gew.-% bis ≤ 85 Gew.-%,
- Pentosane in einer Menge im Bereich von ≥ 0 Gew.-% bis ≤ 10 Gew.-%, bevorzugt ≥ 1 Gew.-% bis ≤ 5 Gew.-%,
- Fette in einer Menge im Bereich von ≥ 2 Gew.-% bis ≤ 10 Gew.-%, bevorzugt ≥ 7 Gew.-% bis ≤ 9 Gew.-%, und
- Asche in einer Menge im Bereich von ≥ 5 Gew.-% bis ≤ 10 Gew.-%, bevorzugt ≥ 7 Gew.-% bis ≤ 9 Gew.-%.

Es konnte gezeigt werden, dass ein Nährmedium mit Arabinoxylan mit einem gewichtsgemittelten Molekulargewicht Mw im Bereich von ≥300 bis ≤500 besonders gut mit der vorbeschriebenen Zusammensetzung herstellbar ist.

Mit der Erfindung wird ferner eine Verwendung des Nährmediums gemäß der Erfindung zur Kultivierung von Pilzen und/oder Hefen vorgeschlagen.

Es konnte überraschend gezeigt werden, dass das gemäß der Erfindung erhaltene Nährmedium hervorragend für die Kultivierung von Pilzen und/oder Hefen geeignet ist. Ohne an eine Theorie gebunden zu sein, wird davon ausgegangen, dass die auf diesem Wege erhaltene Zusammensetzung aus C6-Zuckerfraktion, Pentosanen, Fetten und mineralischen Bestandteilen gute Wachstumsbedingungen ermöglicht, welche die Biomassebildung und Produktivität der Mikroorganismen fördern.

Bevorzugt kann vorgesehen sein, dass die Pilze ausgewählt sind aus essbaren Pilzen, insbesondere aus Pilzen der Gruppe bestehend aus *Agaricus bisporus* (Champignon), *Pleurotus ostreatus* (Austern-Seitling), *Lentinula edodes* (Shiitake), *Pleurotus eryngii* (Kräuterseitling), *Flammulina velutipes* (Enoki), *Grifola frondosa* (Maitake), *Auricularia auricula-judae* (Judasohr), *Ganoderma lucidum* (Reishi), *Macrolepiota procera* (Parasol), *Hydnum repandum* (Semmel-Stoppelpilz), *Kuehneromyces mutabilis* (Stockschwämmchen), *Coprinus comatus* (Schopftintling), *Pholiota nameko* (Goldkäppchen), *Boletus edulis* (Steinpilz), *Cantharellus cibarius* (Pfifferling), *Morchella esculenta* (Morchel), *Tuber* spp. (Trüffel), sowie als Lebensmittel zugelassene Schimmelpilze wie insbesondere *Fusarium venenatum* und *Aspergillus oryzae.*

Es konnte überraschend gezeigt werden, dass das erfindungsgemäße Nährmedium für die Kultivierung essbarer Kulturpilze besonders gut geeignet ist. Insbesondere können mit dem Nährmedium eine stabile Wachstumsleistung und hohe Biomasseausbeuten erzielt werden. Dadurch ist auch die Kultivierung von Pilzen zugänglich, die zuvor nicht oder kaum wirtschaftlich und/oder technisch umsetzbar war. Dies eröffnet insbesondere Anwendungsmöglichkeiten im Bereich der Nahrungsmittelproduktion und der Herstellung von Fleischersatzstoffen.

Bevorzugt kann vorgesehen sein, dass die Pilzen für die Verwendung in Fleischersatzstoffen kultiviert werden.

Es konnte überraschend gezeigt werden, dass das erfindungsgemäße Nährmedium besonders gut für die Kultivierung von Pilzen geeignet ist, die für die Herstellung von Fleischersatzstoffen eingesetzt werden. Insbesondere kann dadurch erreicht werden, dass eine wirtschaftliche und skalierbare Produktion proteinreicher Pilzbiomasse ermöglicht wird, die sensorische und technofunktionelle Eigenschaften aufweist, welche denen von tierischen Produkten ähneln.

Mit der Erfindung wird ferner ein Flüssigfuttermittel vorgeschlagen, welches nach dem erfindungsgemäßen Verfahren hergestellt wurde.

Es konnte überraschend gezeigt werden, dass mit dem erfindungsgemäßen Verfahren neben dem Nährmedium auch ein Flüssigfuttermittel erhalten werden kann, das hinsichtlich seiner Zusammensetzung und Verdaulichkeit gut für die Ernährung von Tieren geeignet ist. Insbesondere kann erreicht werden, dass das Flüssigfuttermittel einen hohen Proteinanteil aufweisen, der in dieser Konzentration bisher aus Nebenströmen der Weizenstärkeproduktion üblicherweise nicht direkt verfügbar war.

Mit der Erfindung wird ein Flüssigfuttermittel vorgeschlagen, wobei das Flüssigfuttermittel bezogen auf das Gesamtgewicht des Flüssigfuttermittels aufweist:
- Wasser in einer Menge im Bereich von ≥ 70 Gew.-% bis ≤ 85 Gew.-%, bevorzugt ≥ 72 Gew.-% bis ≤ 78 Gew.-%,
- Ballaststoffe in einer Menge im Bereich von ≥ 0,1 Gew.-% bis ≤ 10 Gew.-%, bevorzugt ≥ 1 Gew.-% bis ≤ 5 Gew.-%,
- Protein in einer Menge im Bereich von ≥ 10,5 Gew.-% bis ≤ 20 Gew.-%, bevorzugt ≥ 11 Gew.-% bis ≤ 18 Gew.-%,
- Fett in einer Menge im Bereich von ≥ 0,2 Gew.-% bis ≤ 8 Gew.-%, bevorzugt ≥ 2 Gew.-% bis ≤ 6 Gew.-%,
- Kohlenhydrate in einer Menge im Bereich von ≥ 1 Gew.-% bis ≤ 10 Gew.-%, bevorzugt ≥ 2 Gew.-% bis ≤ 8 Gew.-%, und
- Asche in einer Menge im Bereich von ≥ 0,2 Gew.-% bis ≤ 1,7 Gew.-%, bevorzugt ≥ 0,5 Gew.-% bis ≤ 1 Gew.-%.

Es konnte überraschend gezeigt werden, dass ein Flüssigfuttermittel mit dieser Zusammensetzung sich besonders gut als Futtermittel eignet. Ferner konnte gezeigt werden, dass sich diese Zusammensetzung besonders gut mit dem vorliegenden Verfahren herstellen lässt.

Bevorzugt kann vorgesehen sein, dass wobei das Arabinoxylan in dem Verfahren ein gewichtsgemitteltes Molekulargewicht Mw in einem Bereich von ≥ 10 kDa bis ≤ 100 kDa aufweist, und wobei das Flüssigfuttermittel bezogen auf das Gesamtgewicht des Flüssigfuttermittels aufweist:
- Wasser in einer Menge im Bereich von ≥ 70 Gew.-% bis ≤ 85 Gew.-%, bevorzugt ≥ 72 Gew.-% bis ≤ 78 Gew.-%,
- Ballaststoffe in einer Menge im Bereich von ≥ 0,1 Gew.-% bis ≤ 10 Gew.-%, bevorzugt ≥ 1 Gew.-% bis ≤ 5 Gew.-%,
- Protein in einer Menge im Bereich von ≥ 10,5 Gew.-% bis ≤ 20 Gew.-%, bevorzugt ≥ 11 Gew.-% bis ≤ 18 Gew.-%,
- Fett in einer Menge im Bereich von ≥ 0,2 Gew.-% bis ≤ 8 Gew.-%, bevorzugt ≥ 2 Gew.-% bis ≤ 6 Gew.-%,
- Kohlenhydrate in einer Menge im Bereich von ≥ 1 Gew.-% bis ≤ 10 Gew.-%, bevorzugt ≥ 2 Gew.-% bis ≤ 8 Gew.-%, und
- Asche in einer Menge im Bereich von ≥ 0,2 Gew.-% bis ≤ 1,7 Gew.-%, bevorzugt ≥ 0,5 Gew.-% bis ≤ 1 Gew.-%.

Es konnte gezeigt werden, dass ein Flüssigfuttermittel aus dem Verfahren mit Arabinoxylan mit einem gewichtsgemittelten Molekulargewicht Mw im Bereich von ≥ 10 kDa bis ≤ 100 kDa besonders gut mit der vorbeschriebenen Zusammensetzung herstellbar ist.

Bevorzugt kann vorgesehen sein, dass das Arabinoxylan in dem Verfahren ein gewichtsgemitteltes Molekulargewicht in einem Bereich von ≥ 300 kDa bis ≤ 500 kDa aufweist, und wobei das Flüssigfuttermittel bezogen auf das Gesamtgewicht des Flüssigfuttermittels aufweist:
- Wasser in einer Menge im Bereich von ≥ 70 Gew.-% bis ≤ 85 Gew.-%, bevorzugt ≥ 72 Gew.-% bis ≤ 78 Gew.-%,
- Ballaststoffe in einer Menge im Bereich von ≥ 0,1 Gew.-% bis ≤ 5 Gew.-%, bevorzugt ≥ 0,5 Gew.-% bis ≤ 2 Gew.-%,
- Protein in einer Menge im Bereich von ≥ 12 Gew.-% bis ≤ 20 Gew.-%, bevorzugt ≥ 15 Gew.-% bis ≤ 19 Gew.-%,
- Fett in einer Menge im Bereich von ≥ 0,2 Gew.-% bis ≤ 8 Gew.-%, bevorzugt ≥ 2 Gew.-% bis ≤ 6 Gew.-%,
- Kohlenhydrate in einer Menge im Bereich von ≥ 1 Gew.-% bis ≤ 5 Gew.-%, bevorzugt ≥ 1 Gew.-% bis ≤ 2 Gew.-%, und
- Asche in einer Menge im Bereich von ≥ 0,2 Gew.-% bis ≤ 1,7 Gew.-%, bevorzugt ≥ 0,5 Gew.-% bis ≤ 1 Gew.-%.

Es konnte gezeigt werden, dass ein Flüssigfuttermittel aus dem Verfahren mit Arabinoxylan mit einem gewichtsgemittelten Molekulargewicht Mw im Bereich von ≥ 300 bis ≤ 500 besonders gut mit der vorbeschriebenen Zusammensetzung herstellbar ist.

Mit der Erfindung wird ferner eine Arabinoxylanzusammensetzung vorgeschlagen, welche nach dem erfindungsgemäßen Verfahren hergestellt wurde.

Es konnte überraschend gezeigt werden, dass mit dem erfindungsgemäßen Verfahren eine Arabinoxylanzusammensetzung erhalten werden kann, die vergleichsweise einfach mit hoher Reinheit herstellbar ist. Insbesondere kann erreicht werden, dass Arabinoxylane aus Nebenströmen der Weizenstärkeproduktion isoliert und als funktionelle Inhaltsstoffe, etwa für die Tierernährung oder biotechnologische Anwendungen, bereitgestellt werden können.

Mit der Erfindung wird eine Arabinoxylanzusammensetzung vorgeschlagen, wobei die Arabinoxylanzusammensetzung bezogen auf das Gesamtgewicht der Zusammensetzung aufweist:
- Wasser in einer Menge im Bereich von ≥ 4 Gew.-% bis ≤ 10 Gew.-%, bevorzugt ≥ 4,5 Gew.-% bis ≤ 6 Gew.-%,
- Arabinoxylan und/oder Arabinogalactan in einer Menge im Bereich von ≥ 50 Gew.-% bis ≤ 85 Gew.-%, bevorzugt ≥ 65 Gew.-% bis ≤ 80 Gew.-%,
- Protein in einer Menge im Bereich von ≥ 2 Gew.-% bis ≤ 20 Gew.-%, bevorzugt ≥ 10 Gew.-% bis ≤ 18 Gew.-%,
- Fett in einer Menge im Bereich von ≥ 0,1 Gew.-% bis ≤ 6 Gew.-%, bevorzugt ≥ 1 Gew.-% bis ≤ 4 Gew.-%, und
- Asche in einer Menge im Bereich von ≥ 0,1 Gew.-% bis ≤ 1 Gew.-%, bevorzugt ≥ 0,2 Gew.-% bis ≤ 0,7 Gew.-%.

Es konnte gezeigt werden, dass eine Arabinoxylanzusammensetzung mit dieser Zusammensetzung besonders gut mit dem vorliegenden Verfahren herstellen lässt.

### Beispiele

Nachfolgend wird eine bevorzugte technische Lösung anhand bevorzugter Ausführungsbeispiele näher erläutert.

### Beispiel 1

*S. cerevisiae* wurde auf Basis eines erfindungsgemäßen Nährmediums hergestellt. Dabei bestand die C6-Fraktion des Nährmediums aus Glucose und Maltose. Zur Vermeidung der Ethanol-Bildung wurde die Kultivierung im Fed-Batch Verfahren durchgeführt. Hierfür wurden 0,6 m³ einer Hefekultur mit einer Konzentration von 40 kgTM/m³ vorgelegt. Anschließend wurde das erfindungsgemäße Nährmedium (400 kg/m³ Glucose/Maltose) gefüttert. Die Zulaufrate wurde dabei über die CO₂-Konzentration in der Abluft bzw. den Respirationsquotienten geregelt, betrug jedoch im Durchschnitt 0,03 m³/h. Der pH-Wert wurde mit 25%igem Ammoniakwasser und 75%iger Phosphorsäure auf 6,0 gehalten. Die relative Begasungsrate wurde auf 1vvm eingestellt. Nach einer Produktionsdauer von 20h wurde das Endvolumen von 1,2 m³ mit einer Hefekonzentration von 116 kgTM/m³ erreicht. Damit wurde ein Ertrag von 0,48 kgTM/kg Substrat bei einer durchschnittlichen Produktivität von 4,8 kgTM/m³/h erzielt.

### Beispiel 2

*B. edulis* wurde auf Basis eines erfindungsgemäßen Nährmediums hergestellt. Dabei umfasste die C6-Fraktion des Nährmediums mehrheitlich niedermolekulare Dextrine. Die Konzentration des Nährmediums wurde auf 6,4% eingestellt. Anschließend wurde 1m³ des Nährmediums, mit Nährsalzen versetzt, im Bioreaktor sterilisiert und aus einer 150L Vorkultur inokuliert. Die Reaktortemperatur wurde dabei auf 28°C geregelt und der pH-Wert auf 6,5 eingestellt. Die Begasungsrate betrug 1 vvm. Nach einer Batch-Phase von 24h und Erreichen einer Biomassekonzentration von 19,5 kgTM/m³ wurde auf kontinuierliche Betriebsweise umgestellt. Hierfür wurde zuvor sterilfiltriertes Nährmittel mit einer Rate von 0,18 1/h hinzugefügt bzw. entnommen. Gleichzeitig wurde der pH-Wert durch Zugabe von 25%igem Ammoniakwasser konstant gehalten. Die Entnahme des Produktstroms erfolgte mittels Gewichtsregelung über das Bodenablassventil. Die Produktkonzentration im Erntestrom betrug 24,6 kgTM/m³, was einer Ausbeute von 0,48 kgTM/kg Substrat entsprach. Die Produktivität betrug 4,9 kgTM/m³/h.

### Kurze Beschreibung der Zeichnungen

Nachfolgend wird eine bevorzugte technische Lösung unter Bezugnahme auf die anliegenden Zeichnungen anhand bevorzugter Ausführungsbeispiele näher erläutert. Die Formulierung "Figur" ist in den Zeichnungen mit "Fig." abgekürzt.

In den Zeichnungen zeigen
Figur 1 schematisch ein erstes Ausführungsbeispiel eines erfindungsgemäßen Verfahrens, und
Figur 2 schematisch eine Weiterbildung des Ausführungsbeispiels aus Figur 1.

### Detaillierte Beschreibung der Zeichnungen

Die beschriebenen Ausführungsbeispiele sind lediglich Beispiele, die im Rahmen der Ansprüche auf vielfältige Weise modifiziert und/oder ergänzt werden können. Jedes Merkmal, das für ein bestimmtes Ausführungsbeispiel beschrieben wird, kann eigenständig oder in Kombination mit anderen Merkmalen in einem beliebigen anderen Ausführungsbeispiel genutzt werden. Jedes Merkmal, das für ein Ausführungsbeispiel einer bestimmten Anspruchskategorie beschrieben wird, kann auch in entsprechender Weise in einem Ausführungsbeispiel einer anderen Anspruchskategorie eingesetzt werden.

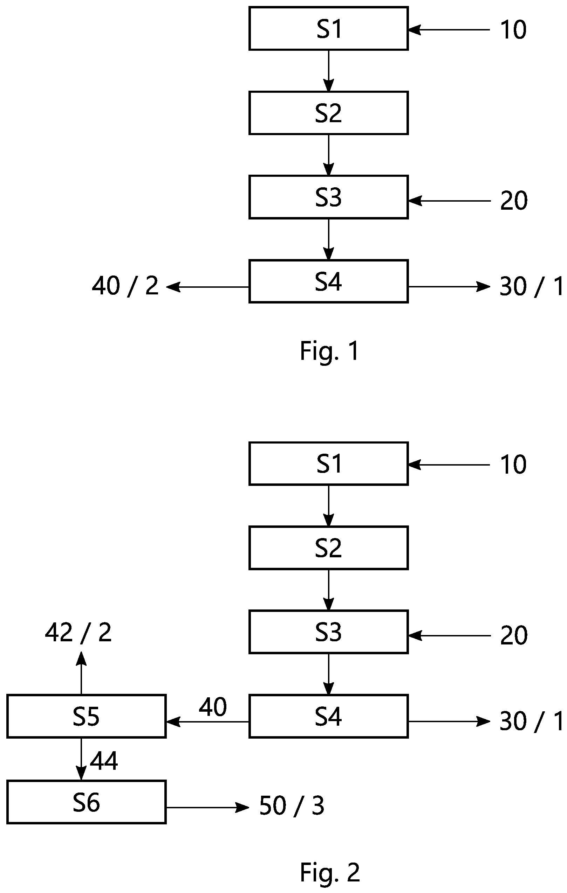

Figur 1 zeigt schematisch ein erstes Ausführungsbeispiel eines erfindungsgemäßen Verfahrens zur Herstellung eines Nährmediums 1. In einem ersten Schritt wird ein Gemisch 10 bereitgestellt S1, bestehend aus Wasser und pflanzlicher Trockenmasse. Anschließend wird das Gemisch 10 erhitzt S2, wodurch eine Hydrolyse eingeleitet wird. Danach wird Amyloglucosidase und/oder alpha-Amylase 20 zugegeben und das Gemisch 10 erneut erhitzt S3, um eine enzymatische Verzuckerung zu bewirken. Das behandelte Gemisch 10 wird sodann filtriert S4, wobei das Filtrat 30 als Nährmedium 1 isoliert wird und das Retentat 40 als Nebenprodukt anfällt, welches als Flüssigfuttermittel isoliert werden kann.

Figur 2 zeigt schematisch eine Weiterbildung des Ausführungsbeispiels aus Figur 1, wobei das Gemisch 10 Arabinoxylan aufweist mit einem gewichtsgemittelten Molekulargewicht Mw in einem Bereich von ≥ 300 kDa bis ≤ 500 kDa. Das Retentat 40 aus Schritt S4 wird dabei in einem weiteren mechanischen Separationsschritt S5 aufgetrennt, wobei das Retentat 42 als Flüssigfuttermittel 2 und das Filtrat 44 isoliert wird. Das Filtrat 44 wird anschließend diafiltriert S6, um ein Retentat 50 zu erhalten, das als Arabinoxylanzusammensetzung 3 isoliert wird.

### Bezugszeichenliste

1 - Nährmedium
2 - Flüssigfuttermittel
3 - Arabinoxylanzusammensetzung
10 - Gemisch
20 - Amyloglucosidase und/oder alpha-Amylase
30 - Filtrat der Filtration S4
40 - Retentat der Filtration S4
42 - Retentat des weiteren mechanischen Separationsschrittes S5
44 - Filtrat des weiteren mechanischen Separationsschrittes S5
50 - Retentat der Diafiltration S6
S1 - Bereitstellen des Gemisches
S2 - Erhitzen des Gemisches
S3 - Zugeben der Amyloglucosidase und/oder alpha-Amylase und Erhitzen
S4 -Filtration
S5 - weitere mechanische Separation
S6 - Diafiltration

## Patentansprüche

1. Verfahren zur Herstellung eines Nährmediums (1), umfassend zumindest die Schritte:
a. Bereitstellen (S1) eines Gemisches (10) bestehend aus Wasser in einem Bereich von ≥ 70 Gew.-% bis ≤ 97 Gew.-% und pflanzlicher Trockenmasse in einem Bereich von ≥ 3 Gew.-% bis ≤ 30 Gew.-%, bezogen auf das Gesamtgewicht des Gemisches (10), wobei die Trockenmasse vergärbare Kohlenhydrate, lösliche Ballaststoffe, Proteine, Fette und Asche umfasst;
b. Erhitzen (S2) des Gemisches (10) auf eine Temperatur im Bereich von ≥ 70 bis ≤ 95°C für einen Zeitraum in einem Bereich von ≥ 0,5 h bis ≤ 2,5 h;
c. Zugeben (S3) einer Amyloglucosidase und/oder alpha-Amylase (20) zu dem Gemisch (10) und Erhitzen des Gemisches auf eine Temperatur im Bereich von ≥ 55 bis ≤ 85°C für einen Zeitraum in einem Bereich von ≥ 0,5 h bis ≤ 8 h;
d. Filtration (S4) des Gemisches (10) mit einer Ultrafiltrationsmembran mit einer Ausschlussgrenze in einem Bereich von ≥ 50 Dalton bis ≤ 200 Dalton und Isolieren des Filtrats (30) der Filtration (S4) als Nährmedium (1).

2. Verfahren nach Anspruch 1, wobei das Gemisch (10) bezogen auf das Gesamtgewicht des Gemisches (10) aufweist:
- Wasser in einer Menge im Bereich von ≥ 72,5 Gew.-% bis ≤ 97 Gew.-%, vorzugsweise ≥ 80 Gew.-% bis ≤ 95 Gew.-%, besonders bevorzugt ≥ 88 Gew.-% bis ≤ 92 Gew.-%;
- vergärbare Kohlenhydrate in einer Menge im Bereich von ≥ 1,75 Gew.-% bis ≤ 21,5 Gew.-%, bevorzugt ≥ 4 Gew.-% bis ≤ 12 Gew.-%, besonders bevorzugt ≥ 6 Gew.-% bis ≤ 8 Gew.-%;
- lösliche Ballaststoffe in einer Menge im Bereich von ≥ 0,25 Gew.-% bis ≤ 2,5 Gew.-%, bevorzugt ≥ 0,5 Gew.-% bis ≤2 Gew.-%, besonders bevorzugt ≥ 1 Gew.-% bis ≤ 1,2 Gew.-%;
- Proteine in einer Menge im Bereich von ≥ 0,3 Gew.-% bis ≤ 6 Gew.-%, bevorzugt ≥ 0,5 Gew.-% bis ≤2 Gew.-%, besonders bevorzugt ≥ 0,8 Gew.-% bis ≤ 1,2 Gew.-%;
- Fette in einer Menge im Bereich von ≥ 0,05 Gew.-% bis ≤ 2 Gew.-%, bevorzugt ≥ 0,3 Gew.-% bis ≤ 1 Gew.-%, besonders bevorzugt ≥ 0,6 Gew.-% bis ≤ 0,8 Gew.-%; und/oder
- Asche in einer Menge im Bereich von ≥ 0,05 Gew.-% bis ≤ 1,3 Gew.-%, bevorzugt ≥ 0,1 Gew.-% bis ≤ 0,5 Gew.-%, besonders bevorzugt ≥ 0,15 Gew.-% bis ≤ 0,25 Gew.-%.

3. Verfahren nach einem der vorherigen Ansprüche, wobei die löslichen Ballaststoffe im bereitgestellten Gemisch (10) Arabinoxylan aufweisen, wobei das Arabinoxylan ein gewichtsgemitteltes Molekulargewicht Mw in einem Bereich von ≥ 300 kDa bis ≤ 500 kDa aufweist oder in einem Bereich von ≥ 10 kDa bis ≤ 100 kDa.

4. Verfahren nach einem der vorhergehenden Ansprüche, wobei in Schritt b) das Gemisch (10) auf eine Temperatur im Bereich von ≥ 75 °C bis ≤ 92 °C, bevorzugt ≥ 80 °C bis ≤ 90 °C, erhitzt wird.

5. Verfahren nach einem der vorhergehenden Ansprüche, wobei in Schritt b) das Gemisch (10) für einen Zeitraum im Bereich von ≥ 0,75 Stunden bis ≤ 2 Stunden, bevorzugt ≥ 1 Stunde bis ≤ 1,5 Stunden, erhitzt wird.

6. Verfahren nach einem der vorhergehenden Ansprüche, wobei in Schritt c) das Gemisch (10) auf eine Temperatur im Bereich von ≥ 55 °C bis ≤ 85 °C, bevorzugt ≥ 60 °C bis ≤ 75 °C, erhitzt wird.

7. Verfahren nach einem der vorhergehenden Ansprüche, wobei in Schritt c) das Gemisch (10) für einen Zeitraum im Bereich von ≥ 0,5 Stunden bis ≤ 8 Stunden, bevorzugt ≥ 1 Stunde bis ≤ 3 Stunden, erhitzt wird.

8. Verfahren nach einem der vorhergehenden Ansprüche, wobei in Schritt d) die Ultrafiltrationsmembran eine Ausschlussgrenze im Bereich von ≥ 75 kDa bis ≤ 150 kDa, bevorzugt ≥ 80 kDa bis ≤ 120 kDa aufweist.

9. Verfahren nach einem der vorhergehenden Ansprüche, wobei in Schritt d) das Retentat (40) der Filtration (S4) als Flüssigfuttermittel (2) isoliert wird.

10. Verfahren nach einem der vorherigen Ansprüche, wobei die löslichen Ballaststoffe im bereitgestellten Gemisch (10) Arabinoxylan aufweisen, wobei das Arabinoxylan ein gewichtsgemitteltes Molekulargewicht Mw in einem Bereich von ≥ 300 kDa bis ≤ 500 kDa aufweist und in Schritt d) das Retentat (40) der Filtration (S4) als Flüssigfuttermittel (2) isoliert wird,
wobei das das Isolieren des Retentats (40) der Filtration (S4) als Flüssigfuttermittel (2) einen Schritt d2) umfasst, in dem ein weiterer mechanischer Separationsschritt (S5) erfolgt, vorzugsweise eine Filtration, wobei das Retentat (42) von Schritt d2) als das Flüssigfuttermittel (2) isoliert wird und das Filtrat (44) von Schritt d2) isoliert wird, wobei das isolierte Filtrat (44) von Schritt d2) des weiteren mechanischen Separationsschrittes (S5) in einem weiteren Schritt d3) mit einer Ultrafiltrationsmembran mit einer Ausschlussgrenze in einem Bereich von ≥ 50 Dalton bis ≤ 200 Dalton im Diafiltrationsmodus filtriert wird (S6) und das Retentat (50) als Arabinoxylanzusammensetzung (3) isoliert wird.

11. Nährmedium (1), hergestellt nach einem Verfahren gemäß einem der Ansprüche 1 bis 10.

12. Nährmedium (1) nach Anspruch 11, wobei das Nährmedium (1) bezogen auf das Gesamtgewicht der Trockenmasse des Nährmediums (1) aufweist:
- eine C6-Zuckerfraktion in einer Menge im Bereich von ≥ 50 Gew.-% bis ≤ 90 Gew.-%, bevorzugt ≥ 65 Gew.-% bis ≤ 80 Gew.-%,
- Pentosane in einer Menge im Bereich von ≥ 0 Gew.-% bis ≤ 20 Gew.-%, bevorzugt ≥ 1 Gew.-% bis ≤ 12 Gew.-%,
- Fette in einer Menge im Bereich von ≥ 0,1 Gew.-% bis ≤ 15 Gew.-%, bevorzugt ≥ 5 Gew.-% bis ≤ 12 Gew.-%, und
- Asche in einer Menge im Bereich von ≥ 0,5 Gew.-% bis ≤ 10 Gew.-%, bevorzugt ≥ 1 Gew.-% bis ≤ 5 Gew.-%.

13. Verwendung des Nährmediums (1) nach einem der Ansprüche 11 und 12, zur Kultivierung von Pilzen und/oder Hefen.

14. Flüssigfuttermittel (2), hergestellt nach einem Verfahren gemäß einem der Ansprüche 9 und 10.

15. Arabinoxylanzusammensetzung (3), hergestellt nach einem Verfahren gemäß Anspruch 10.
